Europäisches Patentamt

(19) European Patent Office

Office européen des brevets

(11) Publication number: **0 027 133**
**B1**

# (12) EUROPEAN PATENT SPECIFICATION

(45) Date of publication of patent specification: **20.02.85**

(51) Int. Cl.⁴: **C 12 Q 1/54, C 12 Q 1/48**

(21) Application number: **80900861.8**

(22) Date of filing: **11.04.80**

(86) International application number:
**PCT/US80/00391**

(87) International publication number:
**WO 80/02296 30.10.80 Gazette 80/25**

(54) **AN IN VITRO METHOD FOR DETERMINING MALIGNANCY IN MAMMALIAN TISSUE.**

(30) Priority: **16.04.79 US 30182**

(43) Date of publication of application:
**22.04.81 Bulletin 81/16**

(45) Publication of the grant of the patent:
**20.02.85 Bulletin 85/08**

(84) Designated Contracting States:
**AT CH DE FR GB LI LU NL SE**

(56) References cited:
**GB-A-1 260 711
GB-A-2 043 890
US-A-3 558 435
US-A-3 616 254
US-A-4 087 331
US-A-4 132 600**

**CHEMICAL ABSTRACTS, vol. 80, no. 3, 21st
January 1974, page 192, no. 12154t, Columbus,
Ohio, U.S.A., N.K. GONATAS et al.: "Detection
of plasma membrane carbohydrates with
lectin peroxidase conjugates"**

(73) Proprietor: **MASSACHUSETTS INSTITUTE OF
TECHNOLOGY**
**77 Massachusetts Avenue**
**Cambridge, MA 02139 (US)**

(72) Inventor: **PLOTKIN, George M.**
**27 Kings Beach Road**
**Lynn MA 01902 (US)**
Inventor: **WOLF, George**
**38 Peacock Farm Road**
**Lexington MA 02173 (US)**

(74) Representative: **Holdcroft, James Gerald, Dr.
et al**
**Graham Watt & Co. Riverhead**
**Sevenoaks Kent TN13 2BN (GB)**

Courier Press, Leamington Spa, England.

(58) References cited:

CHEMICAL ABSTRACTS, vol. 90, no. 7, 12th February 1979, page 443, no. 52857n, Columbus, Ohio, U.S.A., K. HAGEN-COOK et al.: "Galactosyl transferase in benign and neoplastic human bladder mucosa"

CHEMICAL ABSTRACTS, vol. 88, no. 7, 13th February 1978, page 37, no. 48742n, Columbus, Ohio, U.S.A., G.M. PLOTKIN et al.: "Uridine 5'-diphosphate galactose:glycoprotein galactosyl transferase activity in exfoliated bladder epithelial cells in rats fed N-(4-(5-nitro-2-furyl)-2-thiazolyl)-formamide"

NATURE, vol. 256, 21st August 1975, pages 648-650, C.W. PORTER et al.: "Ultrastructural evidence for ectoglycosyl-transferase systems"

CANCER BIOCHEM. BIOPHYS., issued 1977, Plotkin et al., Uridine 5'-diphosphate galactose: glycoprotein galactosyl transferase activity in exfoliated bladder epithelial cells in rats fed N-(4-(5-nitro-2-furyl)-2-thiazolyl) formamide, pages 59-63

BIOCHEM. J., 1975, FRELICH et al., A micro method for simultaneous determination of galactosyltransferase and 5'-nucleotidase activities in cell fractions"

CHEMICAL ABSTRACTS, VOLUME 83:55028j (1975)

CHEMICAL ABSTRACTS, VOLUME 87: 113667n (1977)

CHEMICAL ABSTRACTS, VOLUME 90: 134341d (1979)

CHEMICAL ABSTRACTS, VOLUME 86: 51979j (1977)

CHEMICAL ABSTRACTS, VOLUME 80: 130989s (1974)

CHEMICAL ABSTRACTS, VOLUME 88: 185747g (1978)

## Description

This invention is in the field of biochemistry.

It has been shown that exfoliated bladder cells obtained from the urines of rats fed N-[4-(5-nitro-2-furyl)-2-thiazolyl]foramide for sufficient periods to produce bladder cancer contained significantly increased amounts of the enzyme galactosyl transferase. See Plotkin. G. M. Brigham, S. C., Wolf, G., Jacobs, J. B. and Arai, M., "Uridine 5'-diphosphate galactose: glycoprotein galactosyl transferase activity in exfoliated bladder epithelial cells in rats fed N-[4-(5-nitro-2-furyl)-2-thiazolyl]formamide", *Cancer Biochem. Biophys.*, 1977, Vol. 2., pp59—63. Exfoliated bladder cells obtained from rats similarly treated for much shorter periods of time which are insufficient to cause bladder cancer did not show the significant increase in galactosyl transferase.

Based upon this discovery that the level of a specific enzyme, galactosyl transferase, was significantly different in exfoliated cells from tissue containing cancerous cells than the level in comparable cells for normal tissue, an enzymatic noninvasive test for cancer in mammalian tissue was developed. This test is described in U.S. Patent No. 4,132,600 issued to Plotkin et al. In the method described therein, exfoliated cells from tissue to be tested and assayed for the level of galactosyl transferase activity are incubated with an exogenous galactose acceptor, such as a modified glycoprotein, and a galactose-containing substrate, such as nucleotide galactose sugar. The amount of galactose transferred by the enzyme in a given time under standard conditions to the acceptor can then be determined and is a measure of the level of galactosyl transferase activity present. If the activity is significantly different from normal values, the organ is likely to contain cancerous tissue.

The enzymatic noninvasive method for detecting cancer described in the Plotkin et al patent has proven to be highly reliable.

However the technique of the Plotkin et al. patent is relatively cumbersome involving, as it does, the addition of at least two compounds; a galactose acceptor and a galactose-containing substrate, which is radioactive.

It is an object of the invention to provide a rapid, direct and inexpensive technique for detecting galactose moieties.

We have now found that it is possible to employ only a single exogenous marker material which need not be radioactive.

The present invention provides an *in vitro* method of determining whether mammalian cellular tissue not in suspension in a body fluid therefor is malignant, characterised by adding to the cellular tissue a marker having a specific affinity for galactose moieties, and directly sensing for said marker bound to the cellular tissue.

A method according to the invention comprises, for example, the steps of:

(a) incubating said mammalian cellular tissue in the presence of the marker, which has a specific affinity for galactose moieties present in the membrane of said cells; and

(b) directly sensing for said marker which is bound to said cell membrane.

The present invention also provides an *in vitro* method of determining whether mammalian cellular tissue not in suspension in a body fluid therefor is malignant, characterised by directly detecting the level of galactose moieties exhibited by the plasma membrane of the cellular tissue, and comparing the level found with the level exhibited by non-malignant cellular tissue.

It will be noted in a method according to the invention the galactose moieties, which may be present on or within cells or shed therefrom, are directly sensed or detected, in contrast to the method described in U.S. patent No. 4,132,600, where galactosyl transferase activity is determined.

The marker is for example fluorescent and we have found that while malignant cells exhibit a positive fluorescence for galactose moieties, non-malignant cells exhibit insignificant fluorescence. This is again in contrast to the disclosure of the U.S. patent No. 4,132,600 which shows galactosyl transferase activity in all cells, malignant or non-malignant, the malignant cells giving a quantitative increase in level of galactosyl transferase activity.

In embodiments of the invention the improved method comprises the addition, to cells being tested, of a marker which: (1) has a specific affinity for galactose moieties and (2), once bound to galactose moieties, can be detected because of its inherent optical, electrical, magnetic, or other such properties or because it can be further reacted with materials having such detectable properties. Thus, a chemical assay is not required and a rapid and inexpensive technique for screening a large number of test cells are potentially cancerous is provided.

In the case of bladder cancer, it has been shown that the increased level of galactosyl transferase is predominantly associated with the plasma membrane. This was demonstrated by culturing cells of transitional cell carcinoma line MGH—U1, in suspension, and then assaying them for galactosyl transferase by measurement of the transfer of [³H]galactose from uridine diphosphate-[³H]galactose to desialylated ovine submaxillary mucin. The assay was optimized with respect to time and to protein, uridine diphosphate-galactose, desialylated ovine submaxillary mucin, and Triton X—100 concentrations. This assay was then applied to fresh specimens of benign, inflamed, and neoplastic bladder epithelium from 33 patients who underwent cold-cup biopsies at cystoscopy. Transitional cell carcinoma specimens gave values in the range of 24.7—184.8 cpm [³H]galactose transferred/ug protein per hour (72.0±44.7, mean ± S.D.M., n=25); normal and inflamed specimens ranged from 0.8—46.1 cpm/ug protein per hour (8.3±8.4, means ± S.D.M., n=35). By using a known method of cell rupture, cell ghosts, representing cell-surface membranes, were isolated both from the cultured cell line and from two biopsy specimens of transitional cell carcinoma, and the elevated galactosyl transferase was shown to be located in the cell-surface membrane fraction.

Thus, the improved method of this invention can be employed to detect bladder cancer by assaying for an increased level of galactose moieties in the plasma membrane of bladder cells by introducing a marker compound which has a specific affinity for galactose moieties contained in the plasma membrane of such cells. The marker compound should also be capable of being easily sensed. A marker compound which has both properties can be formed by reacting the lectin Ricinus Communis Agglutinin 120 ($RCA_{120}$), which is derived from Castor beans, with fluorescein, which is a fluorescent dye. This marker compound is commercially available pre-reacted and can be purchased, for example, from Vector Laboratories, Inc., Burlingame, California.

Other lectins, in addition to $RCA_{120}$, can be employed as long as they are specific for galactose moieties. Lectins known to have such specificity and which are commercially available include peanut agglutinin (PNA), Phaseolus Vulgaris Erythro-Agglutinin (PHA—E) and Bandieraea Simplicifolia Lectin (BSL). These lectins are also commercially available from Vector Laboratories.

Of the above-mentioned lectins, $RCA_{120}$ is the preferred lectin because of the very high specificity it displays for galactose moieties.

Similarly, other fluorescent dyes, in addition to fluorescein, can be employed. For example, all fluorescent dyes in the Rhodamine family could be used in place of fluorescein.

Further, the optically detectable marker need not be a fluorescent dye but might have an optical property detectable in some other manner. For example, dyes which produce a direct stain which is detectable could also be bound to a lectin specific for galactose moieties and employed with this invention.

A wide variety of assays employing $RCA_{120}$ bound to fluorescein ($RCA_{120}$—F) have actually been carried out. It has been found that this assay is highly reliable and significantly fluorescent in the plasma membrane for all bladder cells known to be cancerous has been seen. On the other hand, no significant fluorescence has been detected for bladder cells known not to be cancerous.

There is a striking difference between this fluorescence observed in the plasma membrane for cancerous bladder cells contrasted to the lack of any significant fluorescence in the plasma membrane of normal bladder cells.

One or the major advantages of the method described herein is that there is currently existing apparatus capable of detecting optical properties, such as fluorescence, employed with this method. For example, the Ortho Division of Johnson & Johnson produces a Cytofluorograph having this capability. Similarly, Becton and Dickinson markets a Fluoroscence Activated Cell Sorter (FACS) and Coulter Systems markets a Cell Sorter, and both of these can be employed with this invention. Some of these, such as the Cytofluorograph, count the cells present in the sample by sensing scattered light from them while simultaneously counting the number of cells showing fluorescence. Typically, this data is obtained in a histogram which can be obtained in a very short time with a very small sample.

As those skilled in the art will understand, the sensing technique employed to detect the marker compound need not be an optical technique. For example, it may be a ferromagnetic technique, such as nuclear magnetic resonance (NMR) or electron spin resonance (ESR) employed with a marker compound involving ferritin, which has ferromagnetic properties, and which is attached to a compound having a specific affinity for the enzyme. The marker may also be an enzymatic marker and may involve enzymes such as lacto-peroxidase or galactose-oxidase. Such enzymes oxidize galactose which can then be reacted with a chromogen to produce a color.

Radiometric detection procedures are also suitable. For example, galactose moieties in the plasma membrane of cancerous bladder cells could be first oxidized and then labeled with tritium which could be sensed by detecting the radioactive counts produced by a sample.

Radioimmunoassay (RIA) techniques can also be used by employing an antibody coupled to a specific lectin in conjunction with labeled lectin.

Although the experimental work described herein is directed to the detection of bladder cancer, it believed that other forms of cancer can be detected in human or other mammals by the method disclosed. In addition, although the method described herein can usually be employed in a noninvasive mode, it is within the scope of this invention to employ this assay coupled with an invasive method for obtaining the test cells.

This invention can be further and more specifically described by reference to the following examples.

Example 1
Incubation of marker compound with slices of rat tumor and normal bladder tissue

Frozen sections of tumor biopsies from cancerous rat bladder and normal rat bladder tissue were cut using a cryotome. The cancerous tumor was produced by a FANFT-induced AY27 tumor cell line injected into a healthy rat. The biopsies were first frozen in liquid nitrogen or dry ice-acetone, placed in the cryotome and directly cut to 10 um slices. The need for paraffin imbedding was thus obviated.

The tumor and normal slices were subsequently washed and submerged in a solution of $RCA_{120}$—F in phosphate-buffered saline (PBS) for 10 minutes. The solution of marker compound was prepared by dispersing 1 mg of $RCA_{120}$—F obtained from Vector Laboratories in 10 ml of 0.002 M phosphate-buffered saline (PBS), pH 7.2. Slices were removed from the solution, washed once again, and examined with a Leitz UV microscope. Striking fluorescence limited to the cell membrane was observed in the cancerous tissue whereas no fluorescence could be detected for the normal bladder tissue.

Example 2
Marker compound incubated with rat cell lines obtained from bladder tumor
The cancerous rat cell line of Example 1 was employed.

Cells were maintained as monolayer cultures in 35×10 mm falcon plastic Petri dishes with McCoy's 5A medium modification supplemented with 10% fetal calf serum and incubated in 95% air/5% $CO_2$ at 37°C. Cells were harvested near confluency by gentle trypsinisation, washed, resuspended in 0.1 M MES, pH 6.5, and incubated.

Glass cover slips (Fisher 22×22 mm thickness number 1) were placed in a humidified chamber and cells in media were placed on these slides. Cells grew as monolayers over the slides. After a confluent layer had formed over the slides, the slides were washed lightly in PBS in a humidified chamber in 0.5 ml of PBS containing $RCA_{120}$—F, prepared as in Example 1, was placed over the slide and incubated for 10 minutes at 30°C. After the 10-minute incubation at 30° C, the slides were washed gently, again with PBS, and examined with a Leitz UV microscope equipped with bandpass filters for fluorescein. Striking fluorescence limited to the cell membrane was observed.

Example 3
Marker compound incubated with slices of kidney and ureter
The procedures of Example 1 were employed except that the biopsies were taken from normal rat kidney and normal rat ureter. When the slices were examined with a Leitz UV microscope, no significant fluorescence could be detected.

Example 4
Marker compound incubated with human cell lines
The procedures of Example 2 were used, except that four human transitional cell carcinoma cell lines established from human urinary bladder carcinoma at the Massachusetts General Hospital were employed. There were MGH—U1; MGH—U2; RT4; and T24. In addition, an established line derived from normal human urotheliam and the established line MGH—316 derived from normal skin fibroblasts were also employed. Striking fluorescence, limited to the cell membrane, was observed in each tumor cell line, but no significant fluorescence was detected for the normal cell lines.

Example 5
Marker compound incubated with human biopsies
The procedure of Example 1 were employed except that frozen sections of twenty-one bladder tumor biopsies and four normal urothelial biopsies were cut using a cryotome.

Striking fluorescence, limited to the cells membrane, was observed for the cancerous cells, but no significant fluorescence was detected for the normal biopsies.

Example 6
Marker compound incubated with human urine
Urine samples were collected from human patients with and without transitional cell carcinoma. The uring samples were kept at about 30° C and spun in a centrifuge at 1,000×g. The pellets obtained were resuspended in PBS and respun to wash the exfoliated cells. The cells thus obtained were resuspended in PBS, placed on a cover slip and processed with $RCA_{120}$—F as described in Example 2. Upon examination under the Leitz UV microscope, the same striking fluorescence, limited to the cell membrane, was observed for cells from patients having transitional cell carcinoma, but no significant fluorescence was detected for cells from the urine of normal patients.

Example 7
Controls
Glucose, galactose, lactose and concanavalin A lectin were each preincubated at 1, 10 and 100 mM with RCA—F solution described in Example 4. Examples 4, 5 and 6 were then repeated as described.

Glucose had no effect on the results. Galactose and lactose (glucose-galactose) totally inhibited fluorescence at levels tested, presumably by binding to galactose sensitive to RCA lectin. Concanvalin A had no effect on binding of RCA, since it is a lectin specific for glucose and mannose.

**Claims**

1. An *in vitro* method of determining whether mammalian cellular tissue not in suspension in a body fluid therefor is malignant, characterised by adding to the cellular tissue a marker having a specific affinity for galactose moieties, and directly sensing for said marker bound to the cellular tissue.

2. A method according to Claim 1, wherein said marker comprises a lectin bound to a dye.

3. A method according to Claim 2, wherein said dye is a fluorescent dye.

4. A method according to Claim 2 or Claim 3, wherein said lectin comprises $RCA_{120}$.

5. A method according to any one of Claims 2, 3 and 4, wherein said dye comprises fluorescein.

6. A method according to any one of the preceding claims, comprising the steps of:

(a) incubating said mammalian cellular tissue in the presence of the marker, which has a specific affinity for galactose moieties present in the membrane of said cells; and

(b) directly sensing for said marker which is bound to said cell membrane.

7. An *in vitro* method of determining whether mammalian cellular tissue not in suspension in a body fluid therefor is malignant, characterised by directly detecting the level of galactose moieties exhibited by the plasma membrane of the cellular tissue, and comparing the level found with the level exhibited by non-malignant cellular tissue.

8. A method according to Claim 7, wherein the level of galactose moieties is determined by an optical technique.

**Patentansprüche**

1. Verfahren zur Bestimmung in vitro, ob ein Säuger-Zellgewebe, das nicht in zugehöriger Körperflüssigkeit suspendiert ist malignes Gewebe ist, dadurch gekennzeichnet, dass man dem Zellgewebe eine Markierungssubstanz mit spezifischer Affinität zu Galactoseresten zugibt und die an das Zellgewebe gebundene Markierungssubstanz direkt bestimmt.

2. Verfahren nach Anspruch 1, dadurch gekennzeichnet, dass die Markierungssubstanz ein an einen Farbstoff gebundenes Lectin ist.

3. Verfahren nach Anspruch 2, dadurch gekennzeichnet, dass der Farbstoff ein Fluoreszenzfarbstoff ist.

4. Verfahren nach Anspruch 2 oder 3, dadurch gekennzeichnet, dass das Lectin Ricinis Communis Agglutinin 120 ist.

5. Verfahren nach einem der Ansprüche 2, 3, und 4, dadurch gekennzeichnet, dass der Farbstoff Fluorescein ist.

6. Verfahren nach einem der vorstehenden Ansprüche gekennzeichnet durch folgende Schritte:

(a) Inkubation des Säuger-Zellgewebes in Anwesenheit der Markierungssubstanz, die eine spezifische Affinität zu Galaktoseresten besitzt, die sich in der Membran der genannten Zellen befinden; und

(b) Direkbestimmung der an die Zellmembran gebundenen Markierungssubstanz.

7. Verfahren zur Bestimmung in vitro, ob ein Säuger-Zellgewebe, das nicht in zugehöriger Körperflüssigkeit suspendiert ist, malignes Gewebe ist, dadurch gekennzeichnet, dass man die Konzentration der Plasmamembran-Galaktose-reste des Zellgewebes direkt bestimmt und die gefundene Konzentration mit derjenigen von nicht malignem Zellgewebe vergleicht.

8. Verfahren nach Anspruch 7, dadurch gekennzeichnet, dass die Konzentration der Galactosereste mittels einer optischen Arbeitsweise bestimmt wird.

**Revendications**

1. Procédé in vitro pour déterminer si un tissu cellulaire mammifère est malin, ce tissu n'étant pas en suspension dans un liquide corporel naturel du tissu, caractérisé en ce qu'on ajoute au tissu cellulaire un agent de marquage ayant une affinité spécifique à des unités de galactose, et l'on effectue une détection directe de l'agent de marquage lié au tissu cellulaire.

2. Procédé selon la revendication 1, caractérisé que l'agent de marquage est un lectin lié à un colorant.

3. Procédé selon la revendication 2, caractérisé en ce que le dit colorant est un colorant fluorescent.

4. Procédé selon la revendication 2 ou 3, caractérisé en ce que le dit lectin est Ricinus Communis Agglutinin 120.

5. Procédé selon l'une quelconque des revendications 2, 3 et 4, caractérisé en ce que le dit colorant est la fluorescéine.

6. Procédé selon l'une quelconque des revendications précédentes, caractérisé en ce q'il comprend les étapes suivantes:

(a) l'incubation du dit tissu cellulaire mammifère en présence de l'agent de marquage ayant une affinité spécifique à des unités de galactose présentes dans la membrane des dites cellules; et

(b) la détection directe du dit agent de marquage lié à la dite membrane cellulaire.

7. Procédé in vitro pour déterminer si un tissu cellulaire mammifère est malin, ce tissu n'étant pas en suspension dans un liquide corporel naturel du tissu, caractérisé en ce qu'on détermine directement la concentration d'unités de galactose exposées par la membrane plasmique du tissu cellulaire, et l'on compare le concentration trouvée à cell exposée par un tissu cellulaire non-malin.

8. Procédé selon la revendication 7, caractérisé en ce que la concentration des unités de galactose est déterminée par une technique optique.